# EUROPEAN PATENT SPECIFICATION

(11) **EP 1 404 241 B1**
(45) Date of publication and mention of the grant of the patent: **21.06.2006**
(21) Application number: 02741510.8
(22) Date of filing: 24.04.2002
(51) Int. Cl.: A61B 17/70, A61B 17/80

(54) **FIXING DEVICE FOR FIXING VERTEBRA PARTS**
FIXIERVORRICHTUNG ZUR FIXIERUNG VON WIRBELTEILEN
DISPOSITIF POUR FIXATION DE PARTIES DE VERTEBRES

(30) Priority: 24.04.2001 NL 1017932
(43) Date of publication of application: 07.04.2004
(73) Proprietor: De Windt, Paul, Curacao (AN)
(72) Inventor: De Windt, Paul, Curacao (AN)
(74) Representative: van Westenbrugge, Andries
(86) International application number: PCT/NL2002/000270
(87) International publication number: WO 2003/000148

(56) References cited:
- WO-A-99/38447
- DE-U- 9 303 830
- FR-A- 2 766 353
- US-A- 5 147 361
- US-A- 5 616 142
- US-B1- 6 402 756

## Description

The present invention relates to a fixing device according to the preamble of claim 1.

A fixing device of this type is known from US-A-5616142. Two slidable parts are disclosed which can be fastened together fixedly after the sliding piece is fitted into the receiving piece.

From FR 2766353A a fixing device is known, which is used in particular, although not exclusively, for fixing vertebra parts. For all kinds of reasons, it is necessary for vertebrae to be fixed with respect to one another, so that they can grow together. For this purpose, the prior art uses a fixing device comprising fixing plates which are to be secured to each of the vertebra parts.

The fixing plates can move with respect to one another. The fixing plates bear against one another, the lower fixing plate being provided with a screw thread and the upper fixing plate being provided with an opening for receiving a screw. This opening is of elongate design, so that the fixing plates can be adjusted with respect to one another.

It has been found that, under some circurnstances, it is difficult to ensure optimum fixing with a design of this type, since, during surgery, a screwdriver has to be used to exert a considerable force on the screws, and this is not possible under all circumstances. Moreover, in many applications it is desirable to exert a compression on the bone parts in which anchoring is taking place. The bone parts can be made to grow together rapidly as a result of these bone parts being pressed against one another (if appropriate with a further bone part between them). This is not possible with the design shown in FR 2766353.

Moreover, in particular immediately after fitting, there is a risk of the screws becoming loose, with all the associated consequences. Therefore, patients are kept in hospital for a long time and their movement is limited as far as possible, in order to avoid such problems.

The object of the present invention is to provide a more stable structure in which there is less interference with the surrounding tissue. Moreover, a structure of this type must be spatially stable. The rigidity is of particular importance in this context.

In a fixing device as described above, this object is achieved in that the first connecting part comprises two spaced lips for receiving the tongue-like second connecting part in between.

These connecting parts interact with one another as a result of being designed in such a manner that they can slide along one another: This sliding may be a telescopic movement or any other form of sliding movement. In this way, it is possible to obtain a compact structure which projects to a much lesser extent with respect to the bone parts than structures according to the prior art. Firstly, there is less irritation to the surrounding tissue, and secondly the moment which is applied during compression of the bone parts when the fixing plates are being fitted is considerably limited, and in fact simply comprises tension in reaction to the compression.

The connecting parts are designed as a tongue, which is connected to one fixing plate, and two lips, which lie at a distance from one another and are arranged in the second fixing plate, with the tongue sliding between the lips. In the device according to the invention the tongue can be clamped between the lips as a result of a spreading mechanism.

Spreading can be achieved, for example, as a result of conical screws being introduced into openings in the tongue. When they are tightened further, these conical screws press the tongue against the lips so that it is clamped in place. Moreover, the tongue/lips can be roughened in order to improve engagement.

Further stability can be obtained if, in the direction of movement of the connecting parts, the screws lying opposite one another belonging to the different connecting parts are arranged with their ends converging in the bone part in question.

According to an advantageous embodiment, said fixing plates comprise a sleeve-like part which extends beyond them and has a bore with a first diameter which lies in line with an opening in said plate which has a second, larger diameter.

As a result of the openings being designed in a particular way, with the sleeve-like part adjoining them, it is firstly possible to allow screws to be countersunk in the fixing plates. This means that there are no longer any projecting screws, with the result that the surrounding tissue is not irritated. Particularly stable positioning results from the presence of the sleeves which engage in particular in the hard outer layer of the bone of the vertebra part. The screws extend into the softer core part of the bone. The external diameter of sleeves of this type will generally be greater than the diameter of screws which are customary in the prior art, and since they can be of externally smooth design, optimum engagement with the harder part of the bone is ensured.

According to an advantageous embodiment of the invention, sleeves of this type are designed to taper conically on the outside in the direction of the bone. This makes it possible to attain improved fixing in the bone.

It is possible to provide for locking between the screw and the opening in any way which is known from the prior art. For example, it is possible to fit projections/recesses. A design of this type is particularly simple to implement if the sleeve-like part is provided with a screw thread which is used to guide the corresponding screw. After all, when the screw is being screwed in, the force exerted on the screw through engagement with the core material of the bone will be relatively low, and consequently it is not under all circumstances possible to easily feel when the relevant screw is "tight". Providing the sleeve-like part with a screw thread, optionally in combination with an indicator in the vicinity of the opening for the corresponding screw, corresponding to an indicator on the screw itself, allows accurate control of the tightening of the screw in the softer part of the bone.

The invention also relates to a kit comprising a fixing device according to the invention, and screws for securing it, which screws comprise a head with a diameter which is slightly smaller than the said second diameter of the said opening for receiving them.

More particularly, the screws described above are provided with two different screw threads. A first screw thread with a relatively large minor diameter, which is designed to engage on the sleeve-like part, which may optionally be provided with a screw thread. The second screw thread is used to cut into the softer bone material. In this case, the minor diameter is preferably smaller.

The invention will be explained in more detail below with reference to an exemplary embodiment which is illustrated in the drawing, in which:
Fig. 1 shows a perspective, diagrammatic view of the two fixing plates of the fixing device according to the invention in an exploded state;
Fig. 2 shows the way in which bores are made in a vertebra part which is to be provided with the fixing plates according to the invention;
Fig. 3 shows a fixing plate according to the invention arranged on a vertebra part; and
Fig. 4 shows a fixing plate according to the invention arranged on two vertebra parts which lie at a distance from one another; and
Fig. 5 diagrammatically depicts the fixing device according to the invention arranged on two vertebra parts in accordance with the invention.

The fixing device according to the invention is illustrated as an example in the drawings and is denoted overall by 1. It comprises two fixing plates 2, 3 which can be fixed with respect to one another. Each fixing plate comprises a body part 4. Fixing plate 3 is provided with a tongue 5 which projects from the body part as connecting means which is provided with small bores 8 which are adjoined by slots 20. As an alternative to the arrangement shown in the drawing, the end of the tongue may be rounded.

Fixing plate 2 is provided with two lips 6 which lie opposite one another and between which a groove 7 is delimited. The lips are connected beneath the groove by means of a base 21.

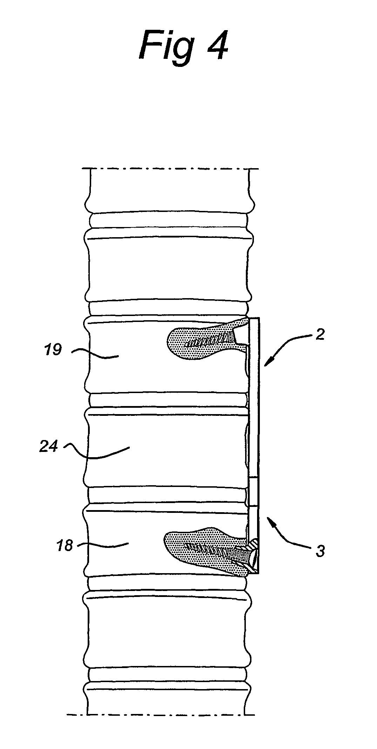

The spacing between the lips 6, i.e. the width of the groove 7, is such that tongue 5 can be received in sliding fashion in the groove. If appropriate, tongue 5 and groove 7 are designed in dovetail form, so that these parts can be guided in a very precise manner with respect to one another. Tongue 5 is fixed in groove 7 as a result of a screw 22 being fitted into bore 8 (Fig. 4), with the result that the slots 20 are spread open, thus producing a clamping action.

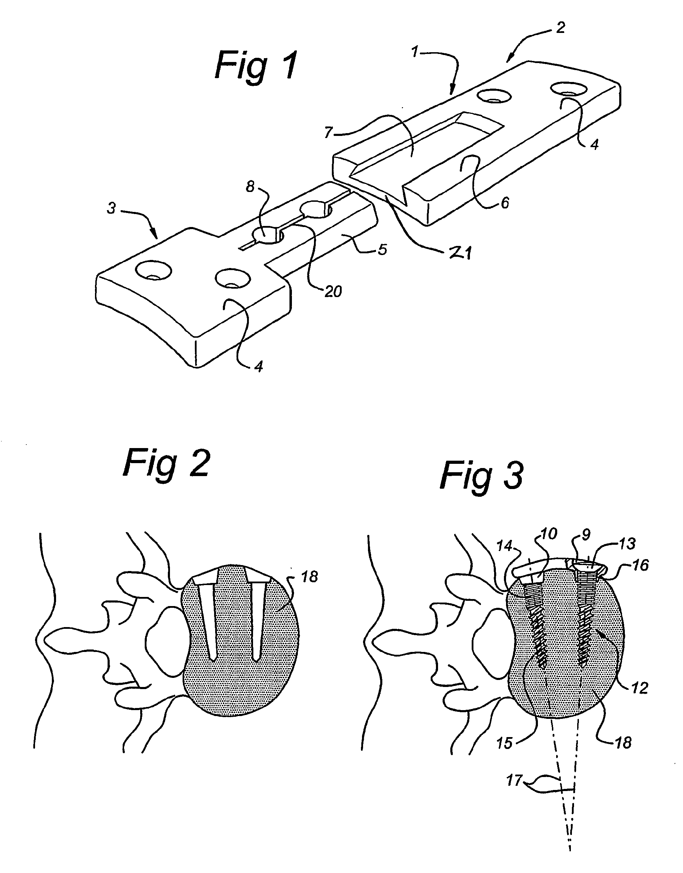

Each of the body parts 4 is provided with two openings. Details of these openings can be seen from Fig. 3. Each opening is denoted by 9 and has a relatively large diameter for receiving the head 13 of a screw 12. Sleeves 10 are present adjacent to each opening 9 and projecting with respect to each fixing plate. These sleeves are designed to taper conically on the outside in the direction away from the plate, i.e. to taper conically towards their free end, and have a smaller bore, so that the head 13 of the screw 12 comes to lie on the top part of the sleeve 10. If appropriate, the sleeves 10 may be provided with a screw thread 16. The centre line of the sleeves is denoted by 17, and as can be seen from Fig. 3, an angle α is delimited between these two centre lines. This angle α is dependent on the application, and in the case of vertebrae, for example, is preferably between 11 and 14°.

Fig. 2 shows the way in which bores are made in a vertebra part 18. They are stepped bores. Bores of this type can be produced in one or two steps using an optionally stepped drill.

After these bores have been made, it is possible to introduce screws 12. These screws 12 are provided with two types of screw thread. A first screw-thread part 14 corresponds to the screw thread 16 in the sleeve part 10 and has a relatively small pitch. A second screw-thread part 15 has a larger pitch and is used for optimum engagement with the relatively soft core material of the vertebra part in question.

The fixing device described above is secured in the following way to the vertebra parts 18 and 19 which are shown in Fig. 4. First of all, the fixing plates 2 and 3 are slid into one another, or are supplied in such a state. Then, they are positioned with respect to one another, in such a way that the corresponding fixing plates can be arranged in the corresponding bores in the vertebrae 18 and the vertebra part 19, respectively. The sleeves 10 provide optimum engagement between the top, widened part of the bores in the vertebra parts, as a result of the conically tapering free ends. Then, the screws 12 are introduced until the relevant fixing plates have been completely fixed. Then, the screws are locked. This can take place in any conceivable way which is known from the prior art. One possibility is for the screws 12 to be provided with a notch, while the edge of the openings 9 is provided with a projection, with the result that a type of snap-in effect is obtained. Another possibility is for the screws 12 to become jammed in a screw thread which is arranged in a corresponding way in the sleeves. Fig. 4 shows a diagrammatic, partial cross section illustrating that the free ends of screws belonging to opposite fixing plates point towards one another. This allows a relatively high compression force to be absorbed.

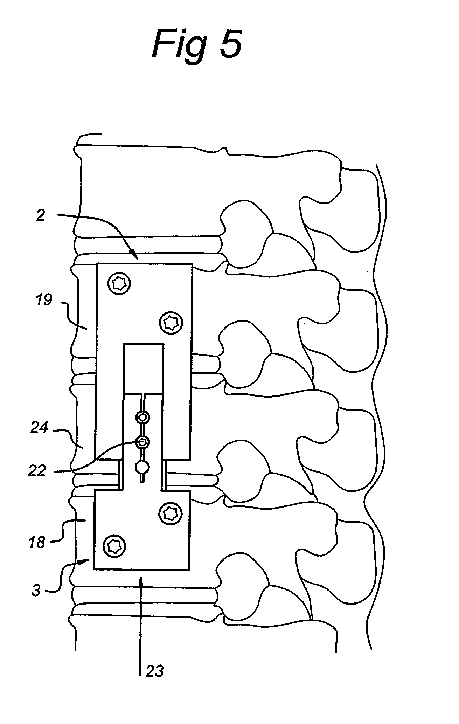

External compression of this type is applied after the fixing plates 2 and 3 have been secured, as illustrated in Fig. 5.

When sufficient force is applied, screw 22 is introduced, with the result that tongue 5 is clamped against lips 6. Then, the external compression can be removed and the result is an externally smooth assembly, in which the force is transmitted to the wall of the opposite vertebra part at the minimum possible distance from the wall of the corresponding vertebra parts. Particularly stable anchoring of the fixing plate is achieved by the design in accordance with the invention. It will be understood that the construction according to the invention makes it considerably easier to fix the fixing plates with respect to one another. Unlike in the prior art, it is no longer necessary to laboriously fit fixing pins at an operation site which is difficult to reach.

It will also be understood that the screws may comprise any structure which is known in the prior art. It is possible to use screws with a double screw thread. The screws are accurately guided by the bore 11 in sleeve 10.

Simple means can be used to apply the compression forces by traction between the two closest parts of the two fixing plates. This contrasts with the arrangement which is known in the prior art, in which pressure is exerted on the parts of the structure which lie furthest apart.

On reading the above description, the person skilled in the art will actually arrive at variants which are obvious and lie within the scope of the appended claims. Moreover, the invention can be used not only to fix vertebra parts, but also to fix other bone parts.

## Claims

1. Fixing device (1) for fixing two bone parts (18, 19) with respect to one another, which device comprises two fixing plates (2, 3), which are each provided with openings (9) for receiving securing means, such as screws (12) for securing the said fixing plate to the said bone part, as well as connecting means for fixing the said fixing parts with respect to one another, said connecting means comprising a first connecting part, which is connected to the first fixing plate, and a second connecting part, which is connected to the second fixing plate, said first and second connecting parts being slideable and fixable with respect to one another, wherein the first connecting part comprises two spaced lips (6) for receiving the tongue-like second connecting part in between (5), **characterized in that**, said second connecting part is spreadable in order to be clamped between the said lips.

2. Fixing device according to claim 1, in which said fixing plates comprise a sleeve-like part (10) which extends beyond said fixing plates and has a bore (11) with a first diameter which lies in line with an opening (9) in the said plate which has a second, larger diameter.

3. Fixing device according to Claim 2, in which the said sleeve-like part is provided with a screw thread (16).

4. Fixing device according to Claim 2 or 3, in which the centre axes of sleeve-like parts of two fixing plates are designed to extend towards one another in the direction of the free ends of the screws.

5. Fixing device according to one of Claims 2-4, in which the sleeve-like part tapers conically on the outside towards its free end.

6. Fixing device according to one of the preceding claims, in which at least two openings which are provided with a sleeve-like part are arranged on a fixing plate, which openings are not aligned with respect to the centre line which connects the said two fixing plates in the fitted state, the centre lines of the said bores being arranged at an angle with respect to one another in the plane which is perpendicular to the centre line which connects the said two fixing plates in the fitted state.

7. Kit comprising a fixing device according to one of the preceding claims in combination with claim 2, and screws (12) for securing it, which screws comprise a head with a diameter which is slightly smaller than the said second diameter of the said opening for receiving them.

8. Kit according to Claim 7, in which the said screws are provided with a first screw thread (14) in the vicinity of the head, with a relatively large core diameter and a second screw thread (15) in the vicinity of their free end, with a relatively small diameter.

## Patentansprüche

1. Fixiervorrichtung (1) zum Fixieren von zwei Knochenteilen (18, 19) in Bezug zueinander, welche Vorrichtung umfasst: zwei Fixierplatten (2, 3), die jeweils mit Öffnungen (9) zur Aufnahme von Sicherungseinrichtungen, wie z.B. Schrauben (12), versehen sind, um die Fixierplatte an dem Knochenteil zu sichern, sowie Verbindungseinrichtungen zum Fixieren der Fixierplatten in Bezug zueinander, wobei die Verbindungseinrichtungen einen ersten Verbindungsteil, der mit der ersten Fixierplatte verbunden ist, und einen zweiten Verbindungsteil, der mit der zweiten Fixierplatte verbunden ist, umfassen, wobei der erste und zweite Verbindungsteil in Bezug zueinander gleitbar und fixierbar sind, wobei der erste Verbindungsteil zwei im Abstand angeordnete Lippen (6) zur Aufnahme des zungenartigen zweiten Verbindungsteils dazwischen (5) umfasst, **dadurch gekennzeichnet, dass** der zweite Verbindungsteil spreizbar ist, um zwischen den Lippen festgeklemmt zu werden.

2. Fixiervorrichtung nach Anspruch 1, bei der die Fixierplatten einen buchsenartigen Teil (10) umfassen, der sich über die Fixierplatten hinaus erstreckt und eine Bohrung (11) mit einem ersten Durchmesser aufweist, die in einer Linie mit einer Öffnung (9) in der Platte, die einen zweiten größeren Durchmesser aufweist, liegt.

3. Fixiervorrichtung nach Anspruch 2, bei der der buchsenartige Teil mit einem Schraubengewinde (16) versehen ist.

4. Fixiervorrichtung nach Anspruch 2 oder 3, bei der die Mittelachsen von buchsenartigen Teilen von zwei Fixierplatten so konzipiert sind, dass sie in der Richtung der freien Enden der Schrauben auf einander zu verlaufen.

5. Fixiervorrichtung nach einem der Ansprüche 2-4, bei der der buchsenartige Teil auf der Außenseite auf sein freies Ende zu konisch verläuft.

6. Fixiervorrichtung nach einem der vorangehenden Ansprüche, bei der mindestens zwei Öffnungen, die mit einem buchsenartigen Teil versehen sind, auf einer Fixierplatte angeordnet sind, welche Öffnungen in Bezug zur Mittellinie, die die zwei Fixierplatten im montierten Zustand verbindet, nicht fluchten, wobei die Mittellinien der Bohrungen unter einem Winkel in Bezug zueinander in der Ebene angeordnet sind, die zu der Mittellinie senkrecht ist, die die zwei Fixierplatten im montierten Zustand verbindet.

7. Bausatz, umfassend eine Fixiervorrichtung nach einem der vorangehenden Ansprüche in Kombination mit Anspruch 2 und Schrauben (12), um sie zu sichern, welche Schrauben einen Kopf mit einem Durchmesser umfassen, der geringfügig kleiner als der zweite Durchmesser der Öffnung ist, um sie aufzunehmen.

8. Bausatz nach Anspruch 7, bei dem die Schrauben mit einem ersten Schraubengewinde (14) in der Nähe des Kopfes mit einem verhältnismäßig großen Kerndurchmesser und einem zweiten Schraubengewinde (15) in der Nähe ihres freien Endes mit einem verhältnismäßig kleinen Durchmesser versehen sind.

## Revendications

1. Dispositif de fixation (1) pour fixer deux parties d'os (18, 19) l'une par rapport à l'autre, ce dispositif comprenant deux plaques de fixation (2, 3), qui sont chacune munies d'ouvertures (9) pour recevoir des moyens de fixation, tels que des vis (12) pour fixer ladite plaque de fixation à ladite partie d'os, ainsi que des moyens de raccordement pour fixer lesdites parties de fixation l'une par rapport à l'autre, lesdits moyens de raccordement comprenant une première partie de raccordement, qui est raccordée à la première plaque de fixation, et une deuxième partie de raccordement, qui est raccordée à la deuxième plaque de fixation, lesdites première et deuxième parties de raccordement pouvant coulisser et pouvant être fixées l'une par rapport à l'autre, la première partie de raccordement comprenant deux lèvres espacées (6) pour recevoir la deuxième partie de raccordement en forme de languette entre elles (5), **caractérisé en ce que** ladite deuxième partie de raccordement peut s'étaler de façon à être serrée entre lesdites lèvres.

2. Dispositif de fixation selon la revendication 1, dans lequel lesdites plaques de fixation comprennent une partie en forme de manchon (10) qui s'étend au-delà desdites plaques de fixation et qui comporte un perçage (11) avec un premier diamètre qui se trouve aligné avec une ouverture (9) dans ladite plaque, et qui a un deuxième diamètre plus grand.

3. Dispositif de fixation selon la revendication 2, dans lequel ladite partie en forme de manchon comporte un filetage (16).

4. Dispositif de fixation selon la revendication 2 ou 3, dans lequel les axes centraux des parties en forme de manchon des deux plaques de fixation sont conçus de façon à s'étendre l'un vers l'autre dans la direction des extrémités libres des vis.

5. Dispositif de fixation selon l'une des revendications 2 à 4, dans lequel la partie en forme de manchon s'effile de façon conique sur l'extérieur vers son extrémité libre.

6. Dispositif de fixation selon l'une des revendications précédentes, dans lequel au moins deux ouvertures qui sont munies d'une partie en forme de manchon sont réalisées sur une plaque de fixation, ces ouvertures n'étant pas alignées par rapport à la ligne centrale qui relie lesdites deux plaques de fixation dans l'état fixé, les lignes centrales desdits perçages étant agencées selon un certain angle l'une par rapport à l'autre dans le plan qui est perpendiculaire à la ligne centrale qui relie lesdites deux plaques de fixation dans l'état fixé.

7. Ensemble comprenant un dispositif de fixation selon l'une des revendications précédentes en combinaison avec la revendication 2, et des vis (12) pour fixer celui-ci, ces vis comprenant une tête avec un diamètre qui est légèrement inférieur audit deuxième diamètre de ladite ouverture pour recevoir celles-ci.

8. Ensemble selon la revendication 7, dans lequel lesdites vis comportent un premier filetage (14) au voisinage de la tête, avec un diamètre de coeur relativement grand et un deuxième filetage (15) au voisinage de leur extrémité libre, avec un diamètre relativement petit.
